(19) Europäisches Patentamt
European Patent Office
Office européen des brevets

(11) EP 4 269 384 A1

(12) EUROPEAN PATENT APPLICATION

(43) Date of publication:
**01.11.2023 Bulletin 2023/44**

(21) Application number: **22170017.2**

(22) Date of filing: **26.04.2022**

(51) International Patent Classification (IPC):
***C07C 213/10*** *(2006.01)* ***C07C 215/54*** *(2006.01)*
***C07C 217/62*** *(2006.01)*

(52) Cooperative Patent Classification (CPC):
(C-Sets available)
**C07C 217/62; C07C 213/10; C07C 215/54;**
C07B 2200/13 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **KRKA, d.d., Novo mesto**
**8000 Novo mesto (SI)**

(72) Inventors:
- **TIHI, Jaroslav**
**800 Novo mesto (SI)**
- **KRASOVEC, Dusan**
**800 Novo mesto (SI)**
- **BEZENSEK, Jure**
**800 Novo mesto (SI)**
- **CESEK, Anita**
**800 Novo mesto (SI)**
- **ANZIC, Borut**
**800 Novo mesto (SI)**

(74) Representative: **Hoffmann Eitle**
**Patent- und Rechtsanwälte PartmbB**
**Arabellastraße 30**
**81925 München (DE)**

Remarks:
Claims 16 to 18 are deemed to be abandoned due to non-payment of the claims fees (Rule 45(3) EPC).

# (54) METHOD FOR CRYSTALLIZING TAPENTADOL INTERMEDIATE, TAPENTADOL INTERMEDIATE OF HIGH PURITY, METHOD OF MAKING TAPENTADOL AND TAPENTADOL OF HIGH PURITY

(57) In one aspect, the present invention relates to a method for crystallizing (2R,3R)-3-(3-methoxyphenyl)-*N*,*N*,2-trimethylpentan-1-amine or salts thereof (compound I), the method comprising the following steps: (i) mixing compound I with a solvent comprising 2-propanol; (ii) heating the mixture obtained in step (i); (iii) cooling the mixture of step (ii); and (iv) separating the crystals obtained in step (iii). In further aspects, the invention provides (2*R*,3*R*)-3-(3-methoxyphenyl)-*N*,*N*,2-trimethylpentan-1-amine or salts thereof in crystal form at high purity level as well as its use in the manufacture of highly pure tapentadol. By consequence, highly pure tapentadol is also provided in yet another aspect of the invention.

EP 4 269 384 A1

(52) Cooperative Patent Classification (CPC): (Cont.)

C-Sets
**C07C 213/10, C07C 217/62**

## Description

### Technical Field

The present application belongs to the field of pharmaceutical research. It provides new methods for preparing the drug tapentadol and its intermediate at high levels of purity.

### Background of the Invention

Tapentadol is an analgesic drug binding to μ-opioid receptors, which may additionally act as a selective norepinephrine reuptake inhibitor.

Methods for preparing tapentadol are known from EP 0 693 475 A, WO 2004/108658 A, WO 2005/00078 A. These methods involve formation of the intermediate shown below (i.e. (2*R*,3*R*)-3-(3-methoxyphenyl)-*N*,*N*,2-trimethylpentan-1-amine, hereinafter referred to as compound I) and its subsequent conversion into tapentadol

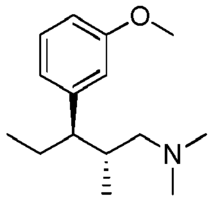

Compound I

US 2009/0312578 A1 describes a further method for obtaining compound I. This document emphasizes the importance of obtaining compound I as a pure stereoisomer since impurities formed by other stereoisomers of compound I will be converted into stereoisomers of tapentadol in a manner that is analogous to the conversion of compound I into tapentadol. These reactions are illustrated by the following reaction schemes:

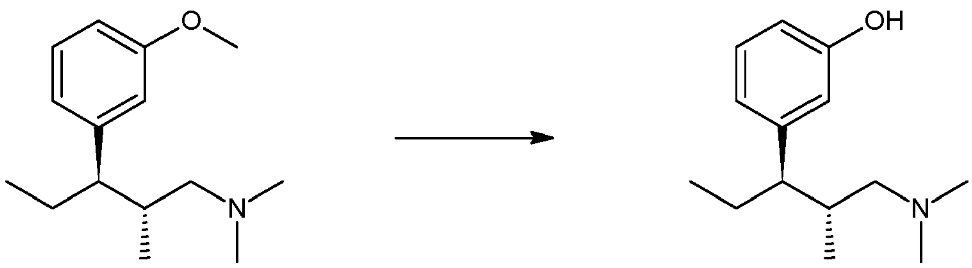

Compound I Tapentadol

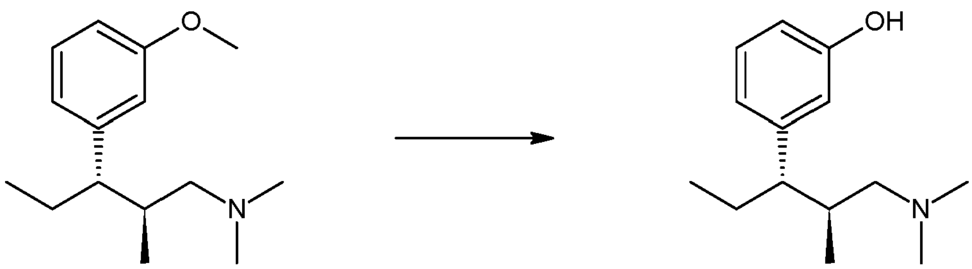

Compound II 2S,3S-Impurity

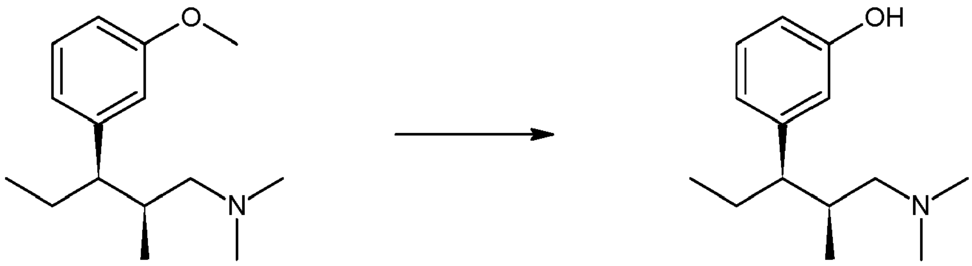

Compound III 2S,3R-Impurity

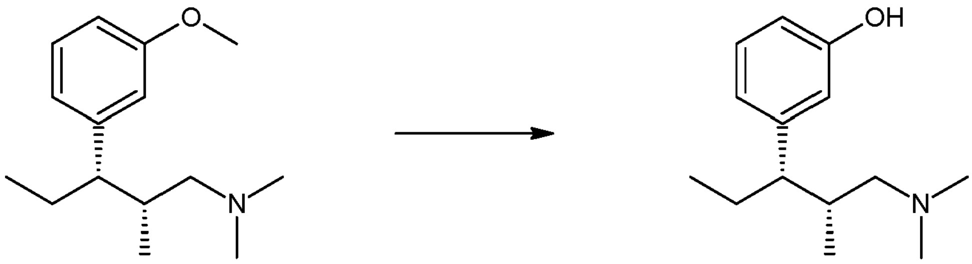

Compound IV 2R,3S-Impurity

Having regard to the general objective of providing tapentadol in highly pure form, the present invention aims to provide methods for purifying compound I by crystallization, which are highly effective in reducing the content of compounds II, III and/or IV.

Another objective of the present invention is to provide a method for preparing highly pure tapentadol, as well as the resulting highly pure tapentadol.

Yet another objective is to provide compounds that can be used as internal or external standards for the determination of purity of compound I and/or tapentadol by chromatographic methods.

**Summary of the Invention**

The objectives outlined above are accomplished by the present invention providing highly efficient methods of purification of compound I by crystallization. As a result of these methods of the invention, compound I in highly purified form is provided as another aspect of the present invention. The purified compound I obtained by the crystallization methods of the present invention may then be used for manufacturing highly pure tapentadol in accordance with yet another aspect of the present invention. Using highly pure compound I as a starting material, it is possible to obtain tapentadol at higher levels of purity. Hence, as a further aspect, the invention also provides tapentadol, which is obtainable by the methods of the present invention and is characterized by a high degree of purity.

Specifically, the present invention provides the methods and products characterized by the following numbered items.

1. Method for crystallizing (2*R*,3*R*)-3-(3-methoxyphenyl)-*N*,*N*,2-trimethylpentan-1-amine or salts thereof (compound I), the method comprising the following steps:

    (i) mixing compound I with a solvent comprising 2-propanol;
    (ii) heating the mixture obtained in step (i);
    (iii) cooling the mixture of step (ii);
    (iv) separating the crystals obtained in step (iii).

2. The method of item 1, wherein the solvent of step (i) consists of 2-propanol or a mixture of 2-propanol and acetone.

3. The method of item 1 or 2, wherein step (ii) is carried out in a closed vessel.

4. The method of any one of items 1 to 3, wherein step (i) and/or step (ii) and/or step (iii) is carried out while stirring.

5. The method of any one of items 1 to 4, wherein step (ii) is carried out until compound I is completely dissolved.

6. The method of any one of items 1, 2, 4 or 5, wherein the mixture in step (ii) is heated to the boiling point of the solvent.

7. The method of item 3, wherein the mixture in step (ii) is heated to a temperature that is from 1°C to 40°C and preferably 10°C to 30°C above the boiling point of the solvent at atmospheric pressure conditions.

8. The method of any one of items 1 to 7, wherein the mixture is cooled in step (iii) to a final temperature of -25°C to 55°C, such as -15°C to 25°C, preferably -8°C to 10°C and more preferably -5°C to 5°C.

9. The method of any one of items 1 to 8, wherein the crystals obtained in step (iv) are subjected to a further step (v): (v) washing the crystals with 2-propanol or acetone, the 2-propanol or acetone having a temperature of 10°C or less, preferably -5°C to +5°C.

10. The method of any one of items 1 to 9, wherein the solvent consists of 2-propanol and the mixing ratio of compound I : 2-propanol is 1:0:0.9 to 1:0:3.0 or 1.0:1.0 to 1.0:3.0, preferably 1.0:1.0 to 1.0:1.7 or 1.0:1.1 to 1.0:1.6, more preferably 1.0:1.2 to 1.0:1.6 by weight/weight, or wherein the solvent consists of a mixture of 2-propanol and acetone and the mixing ratio of compound I : solvent is 1:7 to 1:16 or 1:8 to 1:15 and preferably 1:10 to 1:12 by weight/weight, or the solvent consists of a mixture of 2-propanol and acetone and the mixing ratio of compound I : solvent is 1:3 to 1:7 or 1:3.5 to 1:6 and preferably 1:4 to 1:5, and in particular 1:4.4 by weight/weight.

11. The method of any one of items 2 to 10, wherein the solvent consists of a mixture of 2-propanol and acetone and the mixing ratio of 2-propanol:acetone is 0.5:99.5 to 50:50 or 1:99 to 40:60, preferably 5:95 to 20:80 by weight.

12. The method of any one of items 1 to 11, wherein a step (iii-a) is carried out after step (iii) and before step (iv), wherein
(iii-a) the cooled mixture of step (iii) is stirred at a temperature of -5°C to 10°C and preferably 0°C to 5°C for a time period of 1 min to 3 h and preferably 30 min to 120 min before it is subjected to step (iv).

13. Crystals of (2*R*,3*R*)-3-(3-methoxyphenyl)-*N*,*N*,2-trimethylpentan-1-amine that are obtainable by the method of anyone of items 1 to 12" wherein the content of (2*R*,3*R*)-3-(3-methoxyphenyl)-*N,N*,2-trimethylpentan-1-amine is preferably 99.0 wt.% or more and more preferably 99.5 wt.% or more.

14. The crystals of item 13, wherein the combined content of stereoisomers of tapentadol is 1.0 wt.% or less and preferably 0.5 wt.% or less.

15. Method of preparing tapentadol, which comprises the steps of

(a) providing (2*R*,3*R*)-3-(3-methoxyphenyl)-*N*,*N*,2-trimethylpentan-1-amine or a salt thereof;

(b) subjecting the (2*R*,3*R*)-3-(3-methoxyphenyl)-*N,N*,2-trimethylpentan-1-amine or salt thereof of step (a) to the method according to anyone of items 1 to 12;

(c) subjecting the (2*R*,3*R*)-3-(3-methoxyphenyl)-*N,N*,2-trimethylpentan-1-amine or salt thereof obtained in step (b) to reaction conditions suitable for hydrolysing the methoxy group to yield tapentadol.

16. Method according to item 15, wherein step (c) is carried out under heating, preferably in a temperature range of from 105°C to 110°C and/or preferably in the presence of a catalyst such as HBr.

17. Tapentadol obtainable by the method of item 15 or 16, which preferably is characterized by a purity level of 99.5 wt.% or more.

18. A compound selected from

- 3-(3-methoxyphenyl)-N,N,2-trimethylpropan-1-amine of undefined stereochemistry;
- 3-(3-methoxyphenyl)-N,N,2-trimethylpropan-1-amine in the form of the racemic mixture;

- 3-(3-methoxyphenyl)-N,N,2-trimethylpropan-1-amine in the form of a mixture containing R-enantiomer and S-enantiomer in a ratio of 8.0:2.0 or higher, preferably 9.0:1.0 or higher, more preferably 9.5:0.5 or higher and most preferably 9.9:0.1 or higher;
- 3-(3-methoxyphenyl)-N,N,2-trimethylpropan-1-amine in the form of a mixture containing S-enantiomer and R-enantiomer in a ratio of 8.0:2.0 or higher, preferably 9.0:1.0 or higher, more preferably 9.5:0.5 or higher and most preferably 9.9:0.1 or higher;
- 3-(3-(dimethylamino)-2-methylpropyl)phenol of undefined stereochemistry;
- 3-(3-(dimethylamino)-2-methylpropyl)phenol in the form of the racemic mixture;
- 3-(3-(dimethylamino)-2-methylpropyl)phenol in the form of a mixture containing R-enantiomer and S-enantiomer in a ratio of 8.0:2.0 or higher, preferably 9.0:1.0 or higher, more preferably 9.5:0.5 or higher and most preferably 9.9:0.1 or higher; and
- 3-(3-(dimethylamino)-2-methylpropyl)phenol in the form of a mixture containing S-enantiomer and R-enantiomer in a ratio of 8.0:2.0 or higher, preferably 9.0:1.0 or higher, more preferably 9.5:0.5 or higher and most preferably 9.9:0.1 or higher.

## Detailed Description of the Invention

### *Definitions*

Unless specified otherwise or the context dictates otherwise, all references to compound I are meant to be references to compound I in the free base form and also compound I in the form of a salt thereof.

Unless specified otherwise or the context dictates otherwise, all relative amount indications in % or ppm are to be understood as being based on a weight/weight basis.

In the context of the present invention, the term "comprising" is used to characterize a group of elements such that further unmentioned elements may additionally be present. However, in one specific aspect, the term comprising is intended to characterize a group of elements, which does not include further unmentioned elements. In other words, disclosures with the term "comprising" are to be understood in one aspect as disclosures using the term "consisting of".

Unless specified otherwise or unless the context dictates otherwise, the use of the article "a", "an", etc. is meant to refer to the specified item both in singular and plural form.

Indications of values are to be understood in one aspect as indications of the specified value $\pm$ 10% permitted variation, in another aspect as the indicated value with a permitted variation as determined by conventional rounding rules, and in yet another aspect as the precise specified value without permitted variation.

Any embodiment disclosed herein can be combined with any other embodiment disclosed herein unless explicitly stated otherwise.

When linking two alternative items, the term "and/or" is to be understood as describing either the first item alone, or the second item alone, or a combination of the first and second items.

In the context of the present application, and unless expressly indicated otherwise or the context dictates otherwise, the described operations may be carried out at any temperature including especially room temperature and more specifically a temperature of 22°C. References to room temperature are meant in one embodiment to specify any temperature in the range of 20°C to 25°C. In another embodiment, room temperature is meant to be 22°C.

### *Overview*

The present invention provides a method for crystallizing an intermediate for the synthesis of tapentadol, namely compound I. The key aspect of the present invention is to use 2-propanol as a solvent in the crystallization method. The method thus involves a step of dissolving compound I in the solvent containing or consisting of 2-propanol. This is typically done by mixing compound I with the solvent, followed by heating. After dissolution is accomplished, the solution is cooled to stimulate crystal formation. The solution may be cooled slowly and/or be kept at a constant low temperature to accomplish a high yield of crystallized compound I. The obtained crystals may then be separated, washed and/or dried. The resulting crystals are characterized by a high degree of purity.

Another aspect of the invention relates to compound I of high purity as it can be obtained by the crystallization method of the present invention.

Using such highly pure crystals of compound I in the manufacture of tapentadol permits the formation of highly pure tapentadol. Said manufacture of highly pure tapentadol as well as the resulting highly pure tapentadol product are further aspects of the present invention.

***Crystallization Method***

Starting material

The crystallization method of the present invention uses compound I as a starting material. Said starting material may be provided in solid form, including crystalline or amorphous form. Starting material that is available in dissolved form may also be used. If the solvent is in accordance with the requirements specified hereinbelow, the solution may be used as such which means, in other words, that step (i) may be omitted. If the solvent is not in accordance with the requirements specified hereinbelow, it is possible to provide the starting material in solid form by evaporating the solvent.

The purity of the starting material is not particularly restricted. It may in particular be the purity of the starting material as directly obtained from a suitable process for producing the starting material. Additional purification steps are thus not required. Of course, it is nevertheless possible to subject the starting material to one or more additional purification steps before using it for the method of the present invention. Typical purity levels of the starting material may be in the range of from 50 wt.% to less than 100 wt.%, more specifically 80 wt.% to 99.8 wt.% and even more specifically 90 wt.% to 99.5 wt.% such as 95 wt.% to 99.3 wt.% or 97 wt.% to 99 wt.%.

The starting material may be used in the free base form or in the form of a salt. The hydrochloride salt is preferred but it is equally possible to use alternative salts such as the hydrobromide salt or other salts with inorganic acids.

Solvent

The solvent to be used for the crystallization method of the present invention comprises 2-propanol, sometimes referred to as isopropanol.

In one embodiment, it is possible to use 2-propanol alone as the solvent.

In other embodiments, 2-propanol may be mixed with one or more other solvents to obtain the solvent to be used in the present method. The one or more other solvents are not particularly limited and may be selected from $C_{1-8}$ alcohols other than 2-propanol such as methanol, ethanol, 1-propanol, 1-butanol, 2-butanol, iso-butanol, tert-butanol and the like; linear or cyclic ethers with 2 to 8 carbon atoms and 1 or 2 oxygen atom such as dimethyl ether, diethylether, methyl ethyl ether, tetrahydrofuran, 1,4-dioxane, and the like; ketones with 3 to 6 carbon atoms such as acetone or methyl ethyl ketone; $C_{1-4}$ alkyl esters of $C_{1-4}$carboxylic acids such as ethyl acetate. methanol, ethanol, n-propanol and any type of butanol are most preferred.

The weight ratio of 2-propanol to the combined weight of the one or more other solvents is not particularly restricted and may typically range from 5:95 to 99:1, such as from 10:90 to 98:2, 30:70 to 97:3, 50:50 to 95:5.

In one specific embodiment, the solvent may be composed of a mixture of 2-propanol with acetone. In this embodiment, the weight ratio is also not particularly restricted, ranging from 1:99 to 99:1, but may typically contain larger amounts of acetone. The following typical ranges may be used for the weight ratio of 2-propanol to acetone of from 1:99 to 30:70, 2:98 to 20:80, 5:95 to 15:85 or 7:93 to 12:88. Preferred weight ratios of 2-propanol to acetone are in the range of from 1:8 to 1:12, more preferred 1:9 to 1:11 and most preferred 1:10 (w/w). In further embodiments, the ratio may be characterized by one of the following sub-ranges: 1:99 to 2:98, 2:98 to 5:95; 5:95 to 7:93, 7:93 to 8:92; 8:92 to 9:91, 9:91 to 10:90, 10:90 to 12:88, 12:88 to 15:85, 15:85 to 20:80, 20:80 to 30:70 or 30:70 to 99:1 or any combination of two or more of these ranges.

In further specific embodiments, the solvent may be composed of a mixture of 2-propanol with methyl ethyl ketone. Suitable mixing ratios for the 2-propanol/methyl ethyl ketone embodiment are the same as specified above for the 2-propanol/acetone embodiment.

The purity of the solvent is not particularly restricted. It is, for instance, possible to use industrial technical purity grade, reagent grade or any grade of higher purity. For instance, a purity level of 97 wt.% or higher, 98 wt.% or higher, 99 wt.% or higher, such as 98 wt.% to 99 wt.%, 98.5 wt.% to 99.5 wt.% or 98.0 wt.% to 99.9 wt.% are suitable for use in the present invention.

Vessel

The vessel for carrying out the crystallization method of the present invention is not particularly restricted. The vessel may be provided with a stirring device, a reflux condenser, a device for supplying solids and/or liquids and/or a closure device. The material of the vessel is not particularly restricted and can be, for instance, glass, steel, or a polymer material. If the vessel is provided with a closure device, the material of the vessel must be suited for elevated pressures, such as steel. The size of the vessel must match the batch size of the reaction.

Batch size

The batch size of the crystallization process of the present invention is not particularly limited. It may range from laboratory scale to industrial scale. Typical batch sizes are 10 to 500 kg, preferably 30 to 250 kg and most preferably 50 to 150 kg, especially about 100 kg.

Mixing Step (i)

In the mixing step (i) of the method of the present invention, compound I is combined with the solvent in a suitable container. The relative order of introducing the raw materials into the container is not particularly limited. That is, compound I may be introduced into the container first, followed by addition of the solvent. Alternatively, the solvent may first be introduced into the container, followed by addition of compound I. Likewise, it is possible to simultaneously introduce solvent and compound I.

All substances may be introduced as a single batch or may be introduced in small portions. Again, there is no limitation to the type of addition.

The mixing operation may be carried out under stirring. This can be permanent stirring or intermittent stirring. Stirring may begin together with the beginning of mixing or it may begin after the beginning of mixing.

The type of stirring is not particularly restricted. The stirring equipment is also not particularly restricted. Suitable stirrers include for instance magnetic stirrers, static stirrers, turbines, impellers and the like. Stirring speed is also not particularly restricted. According to one embodiment, no stirring is carried out during the mixing step (i).

The mixing ratio between compound I and solvent depends on the specific nature of the solvent to be used.

If pure 2-propanol is used, a suitable mixing ratio of compound I : 2-propanol is from 1:0:0.9 to 1:0:3.0 or 1.0:1.0 to 1.0:3.0, preferably 1.0:1.0 to 1.0:1.7 or 1.0:1.1 to 1.0:1.6, more preferably 1.0:1.2 to 1.0:1.6 (on a weight/weight basis). In further embodiments, the ratio may be characterized by one of the following sub-ranges: 1.0:0.9 to 1.0:1.0 or 1.0:1.0 to 1.0:1.1 or 1.0:1.1 to 1.0:1.2 or 1.0:1.2 to 1.0:1.5 or 1.0:1.5 to 1.0:1.6, 1.0:1.6 to 1.0:1.7 or 1.7:3.0 or any combination of two or more of these ranges.

If solvent mixtures are used, suitable ranges may vary depending on type and amount of the second solvent.

If the solvent consists of a mixture of 2-propanol and acetone, the mixing ratio of 2-propanol:acetone may be as specified above or is preferably 0.5:99.5 to 50:50 or 1:99 to 40:60, or 5:95 to 20:80 by weight. In further embodiments, the ratio may be characterized by one of the following sub-ranges: 0.5:99.5 to 1:99, 1:99 to 5:95, 5:95 to 20:80, 20:80 to 40:60 or 40:60 to 50:50 or any combination of two or more of these ranges. A specific ratio is 1:10.

In the above embodiments, if a mixture of 2-propanol and acetone, e.g. in a weight/weight ratio of 10:1, is used, compound I and this solvent may be mixed in a weight/weight ratio of 1:7 to 1:16 or 1:8 to 1:15 and preferably 1:10 to 1:12, and in particular 1:11. In further embodiments, the ratio may be characterized by one of the following sub-ranges: 1:7 to 1:8 or 1:8 to 1:10 or 1:10 to 1:11 or 1:11 to 1:12 or 1:12 to 1:15 or 1:15 to 1:16 or any combination of two or more of these ranges.

In an variant of the above embodiments, if a mixture of 2-propanol and acetone e.g. in a weight/weight ratio of 10:1, is used, compound I and this solvent may be mixed in a weight/weight ratio of 1:3 to 1:7 or 1:3.5 to 1:6 and preferably 1:4 to 1:5, and in particular 1:4.4. In further embodiments, the ratio may be characterized by one of the following sub-ranges: 1:3 to 1:4 or 1:4 to 1:5 or 1:5 to 1:6 or 1:6 to 1:7 or any combination of two or more of these ranges.

In specific embodiments, the relative weight ratio of compound I : acetone : 2-propanol may advantageously be 1:10:1 or 1:4:0.4.

If other solvent combinations are used, an appropriate mixing ratio may be determined based on the above information and/or on the basis of simple preliminary experiments. For instance, in one embodiment, the solubility of compound I in 2-propanol and in the solvent mixture of interest may be determined at a temperature 5°C below the boiling temperature of the respective solvent. The appropriate weight or weight fraction of the solvent of interest may then be determined based on the following equation:

$$w(\text{other solvent}) = w(\text{2-propanol}) * [s(\text{2-propanol}) / s(\text{other solvent})]$$

wherein w(other solvent) is the weight or weight fraction of the other solvent;
w(2-propanol) is the weight or weight fraction of the 2-propanol known from the above range indications;
s(other form) is the determined solubility of compound I in the other solvent; and
s(2-propanol) is the determined solubility of compound I in 2-propanol.

For example, if a mixture of 2-propanol with another alcohol ROH is used and if the solubility of compound I in the mixture of 2-propanol and the other alcohol is only half of the solubility of compound I in 2-propanol alone, the weight

fraction for the solvent consisting of a combination of 2-propanol and the other alcohol ROH must be twice as high. Hence, the above range of the ratio of compound I : 2-propanol of 1.0 : 1.0 to 1.0 : 1.3 for 2-propanol translates into a range or ratios of 1.0 : 2.0 to 1.0 : 2.6 for the solvent consisting of 2-propanol and ROH.

The above indications apply to the total weight of compound I in the hydrochloride salt form. If compound I is used in the free base form or in the form of another salt, the above ratio indications need to be converted taking the ratio of molar weight of the hydrochloride salt form of compound I to the molar weight of the form of interest of compound I into account. This can be done relying on the following equation:

$$w(\text{other form}) = w(\text{HCl salt form}) * [M(\text{other form}) / M(\text{HCl salt form})]$$

wherein w(HCl salt form) is the weight or weight fraction of the hydrochloride salt form;
w(other form) is the weight or weight fraction of the other form;
M(HCl salt form) is the molar weight of the hydrochloride salt form; and
M(other form) is the molar weight of the other form.

For instance, a weight ratio of compound I in the HCl form to pure 2-propanol of 1.0 : 1.0 can be converted to a weight ratio of the HBr form based on the following calculation:

$$w(\text{HBr form}) = 1.0 * [302.25 / 257.80] = 1.2$$

with

257.80 being the molar weight of the hydrochloride salt form; and
302.25 being the molar weight of the HBr form.

Hence, a 1.2 : 1.0 weight ratio for the hydrobromide salt corresponds to a 1.0 : 1.0 weight ratio for the hydrochloride salt.

Heating Step (ii)

The mixture of step (i) is heated to a temperature of from 60°C to 100°C, and preferably from 65°C to 90°C, more preferably 70°C to 85°C, such as 75°C to 80°C.

Heating may be carried out in an open vessel with a reflux condenser. In this case, the maximum heating temperature is limited to the boiling point at atmospheric pressure of the employed solvent.

Alternatively, the heating may be carried out in a closed vessel. This allows to increase the temperature beyond the atmospheric pressure boiling point of the solvent.

The heating rate and heating equipment are not particularly limited. Heating rate may depend inter alia on the batch size and heating equipment. Typical heating rates are in the range of from 0.1 °C/min to 20 °C/min and especially 0.5 °C/min to 5 °C/min. There is no particular benefit in increasing or decreasing heating rate as far as product quality is concerned. Hence, an appropriate heating rate may be chosen based on other considerations such as production efficiency and energy consumption.

In one embodiment, the temperature increase by heating may be continued until the entire amount of the compound I is dissolved in the solvent. Complete dissolution may be determined by visual inspection.

The heating step may include one or more time periods of holding the temperature at a constant value (hereinafter "temperature holding period"), including especially the embodiment of holding the temperature at the target heating temperature once this temperature is reached. The duration of such holding periods is not particularly limited and may range from 1 min to 120 min such as 30 min to 90 min and most preferably 60 min. In one embodiment, the temperature is increased until a predetermined temperature within one of the above ranges is reached, followed by a holding period in which the mixture is maintained at this temperature until a clear solution is obtained. Again, it may be confirmed by visual inspection that a clear solution has been formed with no remaining undissolved particles.

Stirring may be carried out during any part of the heating step. As described for the mixing step (i), such stirring can be permanent stirring or intermittent stirring. Stirring may begin with the beginning of heating or it may begin after the beginning of heating. Stirring may also be conducted during the temperature holding period.

The type of stirring is not particularly restricted. The stirring equipment is also not particularly restricted. Suitable stirrers include for instance magnetic stirrers, static stirrers, turbines, impellers and the like. It is particularly advantageous to use the same stirring equipment as in the mixing step (i). Stirring speed is also not particularly restricted. According

to one embodiment, no stirring is carried out during the heating step (ii).

Cooling Step (iii)

In the cooling step, the temperature of the solution obtained in the preceding heating step (ii) is reduced. Cooling may be active cooling that involves contacting the surface of the solution-containing container with a cooling agent. Alternatively, cooling may be passive cooling in which no action is taken other than an interruption of the heating applied during the heating step (ii).

The cooling rate is not particularly limited although cooling rates of from 0.1°C/min to 10 °C/min and preferably 0.5 °C/min to 5. °C/min are advantageously used.

The target temperature of the cooling step (iii) is not particularly limited and may be in the range of from -10°C to 55°C, such as -8°C to 25°C. It may advantageously be within the range of from -5°C to 10°C and preferably 0°C to 5°C.

The cooling step may also include one or more time periods of holding the temperature at a constant value (hereinafter "temperature holding period"), including especially the embodiment of holding the temperature at the target cooling temperature once this temperature is reached. The duration of such holding periods is not particularly limited and may range from 1 min to 120 min such as 30 min to 90 min and most preferably 60 min. In one embodiment, the temperature is decreased until a predetermined target temperature within one of the above ranges is reached, followed by a holding period in which the mixture is maintained at this temperature until a satisfactory amount of crystals is obtained. The formation of crystals may be monitored by visual inspection or IR probe, FBRM (focused beam reflectance measurement) probe, RAMAN probe or on-line camera.

Stirring may be carried out during the cooling step. It may also be carried out during any optionally present temperature holding period. If cooling is carried out, the type of stirring is not particularly restricted. The stirring equipment is also not particularly restricted. Suitable stirrers include for instance magnetic stirrers, static stirrers, turbines, impellers and the like. It is particularly advantageous to use the same stirring equipment as in the preceding step (i) and/or (ii). Stirring speed is also not particularly restricted. According to one embodiment, no stirring is carried out during the cooling step (iii).

Product Isolation Step (iv)

Product isolation may be performed in step (iv) by any known solid/liquid-separation method such as filtering, decantation, and centrifugation.

Optionally, the crystals thus obtained may be washed. The washing liquid to be used for washing is not particularly limited. In one embodiment, 2-propanol is used. To avoid and decide loss of yield, it is advantageous to use a cold washing liquid, for instance having a temperature of -25°C to 10°C, such as -15°C to 10°C and preferably -5°C to 5°C. Depending on the desired degree of purity, the washing operation may be repeated one or more times.

The crystals obtained by the solid/liquid-separation, optionally after washing, may then optionally be dried. Drying can be accomplished by any known solvent evaporation method including especially evaporation by heating and/or one of application of reduced pressure and subjecting the crystals to a stream of gas such as inert gas like nitrogen. Drying may be continued until a predetermined degree of dryness is obtained. This can either be assessed by quantifying the level of remaining solvent or by monitoring weight changes during the drying operation (e.g. such that weight change observed during a predetermined drying period falls below a predetermined threshold level).

Optional further Steps

The procedure described above may be modified by incorporating one or more optional further steps.

This could be, for instance, a step of filtrating the solution obtained in the heating step (ii).

This could also be, for instance, a step of adding seed crystals of compound I at the beginning or during the cooling step (iii).

Possibility to repeat crystallization method

The purity level of compound I of the invention can be further increased if the crystallization method of the invention is carried out two, three or more times, wherein the product of the previous run is used as a starting material for the next run. Hence, in one aspect of the invention, the crystallization method of the invention is repeated once, twice or more often using the product of the previous run as the starting material for the next run.

***Preferred Embodiments***

In the following, preferred embodiments of the above crystallization method are described. The information provided hereinabove and below for the crystallization method of the present invention in its general form applies to these preferred embodiments unless deviating or more specific information is provided hereinbelow.

Preferred Embodiment 1

2-Propanol is used as the only solvent in preferred embodiment 1. In a particularly preferred variant of this embodiment, compound 1 is used in the form of the hydrochloride salt. The mixing ratio of compound I to 2-propanol is 1.0 : 1.0 to 1.0 : 1.6, more preferably 1.0 : 1.1 to 1.0 : 1.6 and most preferably 1.0 : 1.2 to 1.0 : 1.6 (w/w, expressed for the hydrochloride salt of compound I). In further embodiments, the ratio may be characterized by one of the following sub-ranges: 1.0:1.0 to 1.0:1.1 or 1.0:1.1 to 1.0:1.2 or 1.0:1.2 to 1.0:1.5 or 1.0:1.5 to 1.0:1.6 or any combination of two or more of these ranges. Heating is carried out until a target temperature of 80-85°C is reached. As noted above, if an open container is used, the maximum temperature is the boiling point of 2-propanol at atmospheric pressure (82.5°C). A temperature holding period at this temperature is optionally performed. The obtained solution is then cooled to a target temperature of -15°C to 10°C and preferably -5°C to 5°C to yield a suspension of compound I crystals in 2-propanol. Once the target cooling temperature is reached, the suspension is preferably kept at this temperature for a temperature holding period of 30-90 min. The obtained crystals are then isolated by a suitable solid/liquid separation method and preferably the suspension is filtered. Subsequently, the obtained crystals of compound I are preferably washed, in particular with 2-propanol, the 2-propanol being advantageously at a temperature of -5°C to 5°C.

In particularly preferred variants of this embodiment, the mixture is stirred in the heating step and/or in the cooling step including, optionally, stirring during any temperature holding period. Most preferably, stirring is carried out throughout the heating and cooling steps, including any temperature holding period.

Preferred Embodiment 2

In preferred embodiment 2, a mixture of 2-propanol and acetone is used in a mixing ratio of 1:9 to 1:11 and most preferred 1:10 (w/w). This solvent is mixed with compound 1 and preferably compound 1 in the hydrochloride salt form, at a mixing ratio of 1:7 to 1:15, preferably 1:10 to 1:12, and in particular 1:11 (w/w, expressed for the hydrochloride salt of compound I). In further embodiments, the ratio may be characterized by one of the following sub-ranges (w/w): 1:7 to 1:10 or 1:10 to 1:11 or 1:11 to 1:12 or 1:12 to 1:15 or any combination of two or more of these ranges. Heating is carried out in a closed vessel until a target temperature of 75-80°C is reached. A temperature holding period at this temperature is optionally performed. The obtained solution is then cooled to a target temperature of -15°C to 10°C and preferably -5°C to 5°C to yield a suspension of compound I crystals in 2-propanol and acetone. Once the target cooling temperature is reached, the suspension is preferably kept at this temperature for a temperature holding period of 30-90 min. The obtained crystals are then isolated by a suitable solid/liquid separation method and preferably the suspension is filtered. Subsequently, the obtained crystals of compound I are washed, in particular with acetone, the acetone being preferably at a temperature of -5°C to 5°C.

In particularly preferred variants of this embodiment, the mixture is stirred in the heating step and/or in the cooling step including, optionally, stirring during any temperature holding period. Most preferably, stirring is carried out throughout the heating and cooling steps, including any temperature holding period.

Preferred Embodiment 3

In preferred embodiment 3, a mixture of 2-propanol and acetone is used in a mixing ratio of 1:9 to 1:11 and most preferred 1:10 (w/w). This solvent is mixed with compound 1 and preferably compound 1 in the hydrochloride salt form, at a mixing ratio of 1:3 to 1:7, preferably 1:4 to 1:5, and in particular 1:4.4 (w/w, expressed for the hydrochloride salt of compound I). In further embodiments, the ratio may be characterized by one of the following sub-ranges (w/w): 1:3 to 1:4 or 1:4 to 1:5 or 1:5 to 1:6 or 1:6 to 1:7 or any combination of two or more of these ranges. Heating is carried out in a closed vessel until a target temperature of 75-80°C is reached. A temperature holding period at this temperature is optionally performed. The obtained solution is then cooled to a target temperature of -15°C to 10°C and preferably -5°C to 5°C to yield a suspension of compound I crystals in 2-propanol and acetone. Once the target cooling temperature is reached, the suspension is preferably kept at this temperature for a temperature holding period of 30-90 min. The obtained crystals are then isolated by a suitable solid/liquid separation method and preferably the suspension is filtered. Subsequently, the obtained crystals of compound I are washed, in particular with acetone, the acetone being preferably at a temperature of -5°C to 5°C.

In particularly preferred variants of this embodiment, the mixture is stirred in the heating step and/or in the

cooling step including, optionally, stirring during any temperature holding period. Most preferably, stirring is carried out throughout the heating and cooling steps, including any temperature holding period.

***Compound I***

The present invention provides compound I as a product of the crystallization method of the invention. This product is characterized by a higher level of purity resulting from the purification effect of the crystallization method of the invention.

The purity level of compound I of the present invention, as obtained as a product of the crystallization method of the invention, is typically 99.5 weight % or higher, more specifically 99.6 weight % higher, 99.7 weight % higher, 99.8 weight % or higher or even 99.9 weight % higher.

In some aspects, compound I of the present invention is characterized by small residual amounts of impurities: typically, compound II is present in an amount of 0.3 weight % or less, more specifically 0.2 weight % or less or even 0.1 weight % or less.

The combined amount of compounds III and IV is typically 0.4 weight % or less, specifically 0.3 weight % or less, 0.2 weight % or less or even 0.1 weight % or less.

The combined amount of compound II, III and IV is typically 0.5 weight % or less, specifically 0.4 weight % or less, 0.3 weight % or less, 0.2 weight % or less or even 0.1 weight % or less.

The chemical and/or enantiomeric purity level of compound I is to be determined using the following method described in the corresponding section below.

***Method for producing Tapentadol***

The method for producing tapentadol according to the present invention comprises the steps of:

(a) providing (2*R*,3*R*)-3-(3-methoxyphenyl)-*N*,*N*,2-trimethylpentan-1-amine (compound I) or a salt thereof;

(b) subjecting the compound I or salt thereof obtained in step (a) to the crystallization method as described hereinabove and below;

(c) subjecting the compound I or salt thereof obtained in step (b) to reaction conditions suitable for hydrolysing the methoxy group to yield tapentadol.

In the above method for producing tapentadol, all information provided hereinabove and below for the crystallization method can be directly applied to step (b) of the present method. This applies in particular also to the preferred features and combinations of preferred features with other preferred features.

The reaction conditions of step (c) are not particularly restricted as long as they allow a conversion of compound I to tapentadol, i.e. a conversion of the methoxy group to a hydroxy group. In some embodiments, step (c) is carried out under heating, preferably in a temperature range of from 50°C to 120°C, such as 70°C to 90°C, and/or preferably in the presence of a catalyst such as HBr, e.g. in an aqueous solution at a temperature of for instance 105-110°C. Further useful information is found at pages 5 and 23 of WO 2008/012047 A.

***Pure Tapentadol***

The present invention also relates to tapentadol obtainable by the method for producing tapentadol described hereinabove.

The resulting tapentadol is typically highly pure. For instance, it may be characterized by a purity level of 99 wt.% or more, such as 99.5 wt.% or more, 99.8 wt.% or more or even 99.9 wt.% or more. Alternatively, or in addition, the tapentadol obtainable by the method of the present invention is characterized by a content of impurities resulting from compund II of less than 0.15 wt.%, preferably less than 0.10 wt.%. Alternatively, or in addition, the tapentadol obtainable by the method of the present invention is characterized by a combined content of impurities resulting from compounds III and IV of less than 0.10 wt.%, preferably less than 0.05 wt.%.

**Compounds suitable as internal or external standard**

Another embodiment of the present invention relates to the following compound. This is an impurity (hereinafter "impurity 1" or "Imp1") in the product of the method of the present invention. It is 3-(3-methoxyphenyl)-N,N,2-trimethylpropan-1-amine, and which has the following structure:

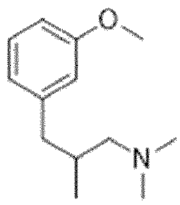

Said impurity 1 is a useful compound as it may be used as an internal or external standard for purity determination of compound I by chromatographic methods such as HPLC. The compound has utility in any defined or undefined stereochemistry, including especially the racemate and mixtures of stereoisomers, wherein one of the enantiomers is enriched such that the ratio of the two enantiomers (i.e. either the (S)/(R)-ratio or the (R)/(S)-ratio) is 8.0:2.0 or higher and preferably 9.0:1.0 or higher, more preferably 9.5:0.5 or higher and most preferably 9.9:0.1 or higher such as a pure enantiomer with no detectable other enantiomer. Another embodiment of the present invention therefore relates to this compound in its various forms.

Likewise, the hydrolysis product of impurity 1, i.e. 3-(3-(dimethylamino)-2-methylpropyl)phenol with the following structure

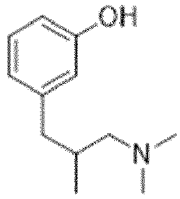

also proves to be useful as an internal or external standard for determining the purity of the hydrolysis product of compound I, i.e. tapentadol, by chromatographic methods such as HPLC. Also this compound has utility in any defined or undefined stereochemistry, including especially the racemate and mixtures of stereoisomers, wherein one of the enantiomers is enriched such that the ratio of the two enantiomers (i.e. either the (S)/(R)-ratio or the (R)/(S)-ratio) is 8.0:2.0 or higher and preferably 9.0:1.0 or higher, more preferably 9.5:0.5 or higher and most preferably 9.9:0.1 or higher such as a pure enantiomer with no detectable other enantiomer. Hence, this compound is the subject of yet another embodiment of the present invention.

**Determination of purity levels**

The **chemical purity** level of compound I is to be determined using the following method:

**Method:**

High-performance liquid chromatography, HPLC on a RP column.

Evaluation method: area %

**Instrument:**

Liquid chromatograph equipped with a gradient pump, UV detector, column thermostat, automated sampling device, and data processing system.

**Standards:**

Compound I (Cpd I) (contains Cpd III/IV in racemic form as well as impurity 1 with undefined stereochemistry)

**Solvents and reagents:**

1. Purified water for chromatography
2. Acetonitrile, $CH_3CN$, for liquid chromatography

3. Methanol, $CH_3OH$, for liquid chromatography
4. Phosphoric acid, $H_3PO_4$, (49-51) %, for liquid chromatography
5. Ammonium dihydrogen phosphate $(NH_4)H_2PO_4$, for liquid chromatography
6. 0.02 M $NH_4H_2PO_4$, pH = 2.4: Weigh about 2.30 g of ammonium dihydrogen phosphate, dissolve in about 950 ml of water and adjust the pH value to 2.4 ± 0.05 with 49-51 % phosphoric acid. Transfer into a 1000 ml volumetric flask and add water to volume.
7. Solvent: 30 % acetonitrile (V/V); Transfer 300 ml of acetonitrile and 700 ml of water using a graduated cylinder into a 1l bottle for the mobile phase, and mix well.

Note: Reagent solution may be prepared with corresponding weight/volume adjustment.

**Chromatographic conditions:**

Column: XSelect CSH C18, (150 x 4.6) mm, 3.5μ μm particles

Note: If necessary, the flow rate or composition of the mobile phase may be slightly adjusted to ensure the suitability of the chromatographic system; a deviation of retention time ± 15% with regard to the retention times at basic conditions is allowed.

**Mobile phase:** A: 0.02 M $NH_4H_2PO_4$, pH = 2.4
B: methanol

**Table 1: Gradient**

| Time (min) | %A | %B | |
|---|---|---|---|
| 0 | 85 | 15 | |
| 15 | 55 | 45 | |
| 23 | 20 | 80 | |
| 31 | 20 | 80 | |
| 32 | 85 | 15 | System equilibration |
| 37 | 85 | 15 | |

**Column temperature:** 60 ºC
**Flow rate:** 1.0 ml/min
**Detection:** UV, 220 nm
**Injection:** 5 μl
**Sampler temperature:** 7 °C

**HPLC solutions::**

Standard/sample solutions can be prepared by corresponding adjustment of weights and volumes to ensure prescribed concentrations of solutions.

**Standard solution of impurity 1 (SS-Imp1)**

Accurately weigh about 10 mg of Imp1 standard into a 20 ml volumetric flask, dissolve in solvent and dilute with solvent to the volume (SS-Imp1).
(SS-Imp1 ~ 0.5 mg/ml

**Solution for system suitability control (SSC)**

Accurately weigh about 10 mg of Cpd I standard for system suitability into a 20 ml volumetric flask, dissolve in 10 ml of solvent, add with pipette 0.2 ml of SS-Imp1 solution and dilute with solvent to the volume (SSC).

**Sample solution (SaS)**

Accurately weigh about 50 mg of sample into a 100 ml volumetric flask, dissolve in solvent and dilute with solvent to the volume (SaS).

(SaS ~ 0.5 mg/ml)

**Procedure**

**A. Chromatographic system suitability**

A1. Inject solvent into an equilibrated chromatographic system.
A2. Inject the solution for selectivity control (SSC) three times.

- Identify Cpd I, Imp1 and Cpd III/IV peaks.
- Determine **relative standard deviation in percent** (RSD %) of Imp1 peak areas.
- Determine **efficiency of the chromatographic column (N)** at Cpd I peak
- Determine **resolution factor (R)** between Cpd I and Cpd III/IV peaks

**Table 2: Acceptance criteria**

| Test | Limit |
|---|---|
| Percentage relative standard deviation (RSD %) of Imp1 peak areas (n=3) | Not more than 2.0 % |
| Efficiency of the chromatographic column (N)) at Cpd I peak. | Not less than 20 000 |
| Resolution factor (R) between Cpd I and Cpd III/IV peaks | Not less than 1.0 |

**B. Analysis**

After testing chromatographic system suitability, inject sample solution (SaS) and follow the response of the main peak and related substances (retention time and area). Chromatographic purity and related substances are evaluated according to the area percentage method. Disregard peaks produced by solvents and peaks smaller than or equal to 0.05 %.

**C. Calculation**

- Chromatographic purity

$$\%\ \text{chromatographic purity (Cpd I)} = A\ (\text{Cpd I}) \times 100 / \Sigma Ai$$

A (Cpd I) = peak area of Cpd I in the chromatogram of the sample solution (SaS)
ΣAi = sum of areas of all integrated peaks in the chromatogram of the sample solution (SaS)

- Related substances - individual:

$$\%\ \text{chromatographic purity (Ai)} = A(Ai) \times 100 / \Sigma Ai$$

A (Ai) = peak area of individual related substance in the chromatogram of the sample solution (SaS)
ΣAi = sum of areas of all integrated peaks in the chromatogram of the sample solution (SaS)

The combined level of compounds III and IV is also determined using the above method, wherein the combined level for compounds III and IV is determined as described above (wherein compounds III and IV appear as a single peak since these compounds are enantiomers and the column is not chiral).
The **enantiomeric purity** of compound I is determined as described in the following.

**Method**

High-performance liquid chromatography, HPLC.
Evaluation method: area %

**Apparatus**

Liquid chromatograph equipped with a gradient pump, variable UV detector, column thermostat, automated sampling device, and data processing system.

**Standard**

Cpd I/II (racemate, contains Cpd I and Cpd II; CAS No.: 809282-11-9)

**Solvents and reagents**

1. n-hexane $C_6H_{14}$, p.a.
2. Methanol, $CH_3OH$, for liquid chromatography
3. Diethylamine (DEA), $C_4H_{11}N$, p.a.
4. n-hexane : DEA = 1000 : 1 (V/V): Transfer 1000 ml of n-hexane using a measuring cylinder into a 1l bottle for the mobile phase, add 1.0 ml of DEA and mix well.
5. Solvent: methanol

Note: If needed, the reagent solutions may be prepared by corresponding weight/volume adjustment.

**Chromatographic conditions**

**Column:** Chiralcel OJ-H, 250 mm x 4.6 mm, 5 μm particles

Note: If necessary, the flow rate or composition of the mobile phase may be slightly adjusted to ensure the suitability of the chromatographic system.

| | |
|---|---|
| Mobile phase: | n-hexane : DEA = 1000 : 1 (V/V) |
| Isocratic elution: | Yes |
| Flow rate: | 0.7 ml/min |
| Detection: | UV, 273 nm |
| Injection: | 2 μl |
| Column temperature: | 40°C |
| Chromatography duration: | 55 minutes* |

*Note: In the case of carry-over peaks, the chromatography duration should be suitably extended.

**HPLC solutions:**

Note: Standard/sample solutions can be prepared by corresponding adjustment of weights and volumes if prescribed concentrations of solutions are provided.

**Solution for system suitability control (SSC)**

Accurately weigh about 50 mg of Cpd I/II into a 5 ml volumetric flask, dissolve and dilute with solvent to the volume (SSC).

**Standard solution (SS)**

Transfer, using a pipette, accurately about 1.0 ml of SSC into a 100 ml volumetric flask, add solvent to the volume, and mix well (SS).

**Sample solution (SaS)**

Accurately weigh about 100 mg of sample into a 10 ml volumetric flask, dissolve and dilute with solvent to the volume (SaS).
SaS: c (sample) ~ 10 mg/ml

**Procedure**

**A. Chromatographic system suitability**

A1. Inject solvent into the equilibrated chromatographic system at least twice.
A2. Inject the standard solution three times (SS).

- Determine relative standard deviation in percent (RSD %) Cpd I peak areas.

A3. Inject once the solution for selectivity control (SSC).
• Identify Cpd I and Cpd II peaks.
• Determine resolution factor (R) between Cpd I and Cpd II peaks.
• Determine efficiency of the chromatographic column (N) at Cpd I peak

**Table 3: Acceptance criteria**

| Test | Limit |
|---|---|
| Resolution factor (R) between Cpd I and Cpd II peaks | Not less than 2.0 |
| Relative standard deviation (RSD %) of Cpd I peak areas (n=3) | Not more than 2.0 % |
| Efficiency of the chromatographic column (N) at Cpd I peak | Not less than 5000 |

**B. Analysis**

After testing chromatographic system suitability, inject sample solution (SaS) and follow the response (retention time and area of the peak) of Cpd I and Cpd II peaks.
Enantiomeric purity is evaluated according to the area percentage method. Only Cpd I and Cpd II peaks are integrated. Disregard Cpd II peaks smaller than or equal to 0.05 %.

**C. Calculation**

- Enantiomeric purity:

$$\%Cpd\ I = A\ (Cpd\ I) \times 100 / \Sigma Ai$$

A (Cpd I) = Cpd I peak area in the chromatogram of the sample solution (SaS)

Σ Ai = sum of areas of both integrated peaks (Cpd I and II) in the chromatogram of the sample solution

The calculation for compound II is done in an analogous manner.

The chemical and/or enantiomeric purity level of tapentadol is to be determined by analogous application of the procedures described above for compound I.

While the invention is described hereinabove and below in terms of various specific embodiments and different aspects, it will be apparent to the person skilled in the art that various modifications and improvements may be made without departing from the scope of the invention as defined by the appended claims. Accordingly, the invention is not to be limited by the specific embodiments and aspects described herein.

## Examples

Experimental protocol 1

10 g of Compound I hydrochloride is mixed with 15 ml of 2-propanol. The mixture is then heated to reflux temperature and kept at this temperature under stirring. After all of compound I is dissolved, the solution is cooled to 0 to 5°C and stirred for another one hour. The suspension is filtered and compound I hydrochloride is rinsed with cold 2-propanol.

Experimental protocol 2

14g of compound I hydrochloride is suspended in a mixture of acetone and 2-propanol. The weight:weight:weight ratio of compound I hydrochloride : acetone : 2-propanol is 1 : 4 : 0.4. The reaction vessel is tightly closed and the suspension is heated to 75 to 80°C. Under this condition the overpressure in the reaction vessel is up to 2 bars. After no particles are observed the reaction mixture is cooled to the cooling target temperature specified in the table below and stirred for another hour. The product is filtered and washed with cold acetone.

In the variant 2a of this experimental protocol, the same procedure was applied but the ratio of compound I hydrochloride : acetone : 2-propanol is 1:10 : 1.

The above experimental protocols are carried out using different starting material qualities. The results of these experiments are summarized in the following table.

**Table 4: Experimental results**

| | Experimental Protocol | Cooling Target temperature | Compound I | Compound II* | Mixture of Compound III and IV |
|---|---|---|---|---|---|
| Sample 1 prior to crystallization | | | 99.00 % | 0,79 % | 0.78 % |
| | | | | | |
| Sample 1 after crystallization | 1 | 0-5°C | 99.62 % | 0.09% | 0.26 % |
| | | | | | |
| Sample 1 after crystallization | 2 | 0-5°C | 99.78 % | 0.04 % | 0.15 % |
| | | | | | |
| Sample 2 prior to crystallization | | | 99.18 | | 0.54% |
| Sample 2 after crystallization | 2 | 0°C | 99.63% | | 0.31% |
| Sample 2 after crystallization | 2 | 20-25°C | 99.62% | | 0.30% |
| Sample 2 after crystallization | 2 | 50°C | 99.66% | | 0.28% |
| | | | | | |

(continued)

| | Experimental Protocol | Cooling Target temperature | Compound I | Compound II* | Mixture of Compound III and IV |
|---|---|---|---|---|---|
| Sample 3 prior to crystallization | | | 99.75 % | | 0.77%** |
| Sample 3 after crystallization | 2 | 0°C | 99.82 % | 0.002% | 0.09%<br>0.125 %** |
| | | | | | |
| Sample 4 prior to crystallization | | | 99.55 % | 0.62 % | 0.61 %** |
| Sample 4 after crystallization | 2a | 0°C | 99.84 % | 0.02 % | 0.15 %** |
| *For this impurity analysis with chiral chromatography was used<br>** Mixture of Compound III and IV were determined by GC chromatography | | | | | |

## Claims

1. Method for crystallizing (2*R*,3*R*)-3-(3-methoxyphenyl)-*N*,*N*,2-trimethylpentan-1-amine or salts thereof (compound I), the method comprising the following steps:

   (i) mixing compound I with a solvent comprising 2-propanol;
   (ii) heating the mixture obtained in step (i);
   (iii) cooling the mixture of step (ii);
   (iv) separating the crystals obtained in step (iii).

2. The method of claim 1, wherein the solvent of step (i) consists of 2-propanol or a mixture of 2-propanol and acetone.

3. The method of claim 1 or 2, wherein step (ii) is carried out in a closed vessel.

4. The method of any one of claims 1 to 3, wherein step (i) and/or step (ii) and/or step (iii) is carried out while stirring.

5. The method of any one of claims 1 to 4, wherein step (ii) is carried out until compound I is completely dissolved.

6. The method of any one of claims 1, 2, 4 or 5, wherein the mixture in step (ii) is heated to the boiling point of the solvent.

7. The method of claim 3, wherein the mixture in step (ii) is heated to a temperature that is from 1°C to 40°C and preferably 10°C to 30°C above the boiling point of the solvent at atmospheric pressure conditions.

8. The method of any one of claims 1 to 7, wherein the mixture is cooled in step (iii) to a final temperature of -25°C to 55°C, such as -15°C to 25°C, preferably -8°C to 10°C and more preferably -5°C to 5°C.

9. The method of any one of claims 1 to 8, wherein the crystals obtained in step (iv) are subjected to a further step (v): (v) washing the crystals with 2-propanol or acetone, the 2-propanol or acetone having a temperature of 10°C or less, preferably -5°C to +5°C.

10. The method of any one of claims 1 to 9, wherein the solvent consists of 2-propanol and the mixing ratio of compound I : 2-propanol is 1:0:0.9 to 1:0:3.0 or 1.0:1.0 to 1.0:3.0, preferably 1.0:1.0 to 1.0:1.7 or 1.0:1.1 to 1.0:1.6, more preferably 1.0:1.2 to 1.0:1.6 by weight/weight, or wherein the solvent consists of a mixture of 2-propanol and acetone and the mixing ratio of compound I : solvent is 1:7 to 1:16 or 1:8 to 1:15 and preferably 1:10 to 1:12 by weight/weight, or the solvent consists of a mixture of 2-propanol and acetone and the mixing ratio of compound I : solvent is 1:3 to 1:7 or 1:3.5 to 1:6 and preferably 1:4 to 1:5, and in particular 1:4.4 by weight/weight.

**11.** The method of any one of claims 2 to 10, wherein the solvent consists of a mixture of 2-propanol and acetone and the mixing ratio of 2-propanol:acetone is 0.5:99.5 to 50:50 or 1:99 to 40:60, preferably 5:95 to 20:80 by weight.

**12.** The method of any one of claims 1 to 11, wherein a step (iii-a) is carried out after step (iii) and before step (iv), wherein (iii-a) the cooled mixture of step (iii) is stirred at a temperature of -5°C to 10°C and preferably 0°C to 5°C for a time period of 1 min to 3 h and preferably 30 min to 120 min before it is subjected to step (iv).

**13.** Crystals of (2*R*,3*R*)-3-(3-methoxyphenyl)-*N*,*N*,2-trimethylpentan-1-amine that are obtainable by the method of anyone of claims 1 to 12" wherein the content of (2R,3R)-3-(3-methoxyphenyl)-*N,N*,2-trimethylpentan-1-amine is preferably 99.0 wt.% or more and more preferably 99.5 wt.% or more.

**14.** The crystals of claim 13, wherein the combined content of stereoisomers of tapentadol is 1.0 wt.% or less and preferably 0.5 wt.% or less.

**15.** Method of preparing tapentadol, which comprises the steps of

(a) providing (2*R*,3*R*)-3-(3-methoxyphenyl)-*N*,*N*,2-trimethylpentan-1-amine or a salt thereof;
(b) subjecting the (2*R*,3*R*)-3-(3-methoxyphenyl)-*N,N*,2-trimethylpentan-1-amine or salt thereof of step (a) to the method according to anyone of claims 1 to 12;
(c) subjecting the (2*R*,3*R*)-3-(3-methoxyphenyl)-*N,N*,2-trimethylpentan-1-amine or salt thereof obtained in step (b) to reaction conditions suitable for hydrolysing the methoxy group to yield tapentadol.

**16.** Method according to claim 15, wherein step (c) is carried out under heating, preferably in a temperature range of from 105°C to 110°C and/or preferably in the presence of a catalyst such as HBr.

**17.** Tapentadol obtainable by the method of claim 15 or 16, which preferably is **characterized by** a purity level of 99.5 wt.% or more.

**18.** A compound selected from

- 3-(3-methoxyphenyl)-N,N,2-trimethylpropan-1-amine of undefined stereochemistry;
- 3-(3-methoxyphenyl)-N,N,2-trimethylpropan-1-amine in the form of the racemic mixture;
- 3-(3-methoxyphenyl)-N,N,2-trimethylpropan-1-amine in the form of a mixture containing R-enantiomer and S-enantiomer in a ratio of 8.0:2.0 or higher, preferably 9.0:1.0 or higher, more preferably 9.5:0.5 or higher and most preferably 9.9:0.1 or higher;
- 3-(3-methoxyphenyl)-N,N,2-trimethylpropan-1-amine in the form of a mixture containing S-enantiomer and R-enantiomer in a ratio of 8.0:2.0 or higher, preferably 9.0:1.0 or higher, more preferably 9.5:0.5 or higher and most preferably 9.9:0.1 or higher;
- 3-(3-(dimethylamino)-2-methylpropyl)phenol of undefined stereochemistry;
- 3-(3-(dimethylamino)-2-methylpropyl)phenol in the form of the racemic mixture;
- 3-(3-(dimethylamino)-2-methylpropyl)phenol in the form of a mixture containing R-enantiomer and S-enantiomer in a ratio of 8.0:2.0 or higher, preferably 9.0:1.0 or higher, more preferably 9.5:0.5 or higher and most preferably 9.9:0.1 or higher; and
- 3-(3-(dimethylamino)-2-methylpropyl)phenol in the form of a mixture containing S-enantiomer and R-enantiomer in a ratio of 8.0:2.0 or higher, preferably 9.0:1.0 or higher, more preferably 9.5:0.5 or higher and most preferably 9.9:0.1 or higher.

Europäisches Patentamt
European Patent Office
Office européen des brevets

# EUROPEAN SEARCH REPORT

Application Number
EP 22 17 0017

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2009/312578 A1 (FILLIERS WALTER FERDINAND MARI [BE] ET AL) 17 December 2009 (2009-12-17)<br>* paragraphs [0027], [0040] - [0042] *<br>* paragraph [0002] *<br>* abstract *<br>----- | 1-15 | INV.<br>C07C213/10<br>C07C215/54<br>C07C217/62 |
| A | WO 2011/128784 A2 (ACTAVIS GROUP PTC EHF [IS]; KHUNT MAYUR DEVJIBHAI [IN] ET AL.) 20 October 2011 (2011-10-20)<br>* paragraphs [0011] - [0012] *<br>* paragraph [0099] *<br>* examples 9-14 *<br>* claims 9-11 *<br>----- | 1-15 | |

TECHNICAL FIELDS SEARCHED (IPC)
C07C

The present search report has been drawn up for all claims

1

| Place of search | Date of completion of the search | Examiner |
|---|---|---|
| The Hague | 23 January 2023 | Fitz, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 17 0017

This annex lists the patent family members relating to the patent documents cited in the above-mentioned European search report. The members are as contained in the European Patent Office EDP file on The European Patent Office is in no way liable for these particulars which are merely given for the purpose of information.

23-01-2023

| Patent document cited in search report | Publication date | Patent family member(s) | Publication date |
|---|---|---|---|
| US 2009312578 A1 | 17-12-2009 | AT 455752 T | 15-02-2010 |
| | | AU 2007278224 A1 | 31-01-2008 |
| | | BR PI0714671 A2 | 07-05-2013 |
| | | CA 2656696 A1 | 31-01-2008 |
| | | CN 101495447 A | 29-07-2009 |
| | | CY 1110075 T1 | 14-01-2015 |
| | | DK 2046726 T3 | 10-05-2010 |
| | | EA 200970147 A1 | 28-08-2009 |
| | | EP 2046726 A1 | 15-04-2009 |
| | | ES 2339403 T3 | 19-05-2010 |
| | | HK 1134280 A1 | 23-04-2010 |
| | | HR P20100197 T1 | 31-05-2010 |
| | | IL 196627 A | 27-06-2013 |
| | | JP 5208933 B2 | 12-06-2013 |
| | | JP 2009544658 A | 17-12-2009 |
| | | KR 20090033444 A | 03-04-2009 |
| | | PL 2046726 T3 | 30-06-2010 |
| | | PT 2046726 E | 15-04-2010 |
| | | SI 2046726 T1 | 31-05-2010 |
| | | US 2009312578 A1 | 17-12-2009 |
| | | US 2012142970 A1 | 07-06-2012 |
| | | WO 2008012283 A1 | 31-01-2008 |
| WO 2011128784 A2 | 20-10-2011 | AU 2011241897 A1 | 27-09-2012 |
| | | CA 2793948 A1 | 20-10-2011 |
| | | EP 2556048 A2 | 13-02-2013 |
| | | US 2013096347 A1 | 18-04-2013 |
| | | WO 2011128784 A2 | 20-10-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0693475 A
- WO 2004108658 A
- WO 200500078 A
- US 20090312578 A1
- WO 2008012047 A

### Non-patent literature cited in the description

- *CHEMICAL ABSTRACTS,* 809282-11-9